# EUROPEAN PATENT SPECIFICATION

(11) **EP 4 380 521 B1**
(45) Date of publication and mention of the grant of the patent: **06.05.2026**
(21) Application number: 22748444.1
(22) Date of filing: 13.07.2022
(51) Int. Cl.: A61F 9/007

(54) **MANAGING PHACOEMULSIFICATION USER DEFINED PROTOCOLS**
VERWALTUNG VON BENUTZERDEFINIERTEN PROTOKOLLEN FÜR PHAKOEMULSIFIKATION
GESTION DE PROTOCOLES DÉFINIS PAR UN UTILISATEUR POUR LA PHACOÉMULSIFICATION

(30) Priority: 07.08.2021 US 202163230741 P; 10.05.2022 US 202217740685
(43) Date of publication of application: 12.06.2024
(73) Proprietor: Johnson & Johnson Surgical Vision, Inc., Irvine, CA 92618 (US)
(72) Inventor: GOVARI, Assaf, 2066717 Yokneam (IL); BEECKLER, Christopher Thomas, Irvine, California 92618 (US); GLINER, Vadim, 2066717 Yokneam (IL); SITNITSKY, Ilya, 2066717 Yokneam (IL); FUCHS, Amit, 2066717 Yokneam (IL); KEYES, Joseph Thomas, Irvine, California 92618 (US); AHARON, Eran, 2066717 Yokneam (IL)
(74) Representative: Carpmaels & Ransford LLP
(86) International application number: PCT/IB2022/056469
(87) International publication number: WO 2023/017335

(56) References cited:
- EP-A2- 0 956 840
- WO-A1-2021/119616
- US-A1- 2006 129 140
- US-A1- 2007 161 972
- US-A1- 2014 114 296

## Description

### FIELD OF THE DISCLOSURE

The present disclosure relates generally to eye surgery systems, and particularly to user interfaces and algorithms to manage user defined phacoemulsification protocols.

### BACKGROUND OF THE DISCLOSURE

A cataract is a clouding and hardening of the eye's natural lens, a structure which is positioned behind the cornea, iris and pupil. The lens is mostly made up of water and protein and as people age these proteins change and may begin to clump together obscuring portions of the lens. To correct this, a physician may recommend phacoemulsification cataract surgery. In the procedure, the surgeon makes a small incision in the sclera or cornea of the eye. Then a portion of the anterior surface of the lens capsule is removed to gain access to the cataract. The surgeon then uses a phacoemulsification probe, which has an ultrasonic handpiece with a needle. The tip of the needle vibrates at ultrasonic frequency to sculpt and emulsify the cataract while a pump aspirates particles and fluid from the eye through the tip. Aspirated fluids are replaced with irrigation of a balanced salt solution to maintain the anterior chamber of the eye. After removing the cataract with phacoemulsification, the softer outer lens cortex is removed with suction. An intraocular lens (IOL) is then introduced into the empty lens capsule restoring the patient's vision.

The disclosure of US2014/114296A1 provides methods and systems for planning and forming incisions in a cornea, lens capsule, and/or crystalline lens nucleus. A method includes measuring spatial dispositions, relative to a laser surgery system, of at least portions of the corneal anterior and posterior surfaces. A spatial disposition of an incision of the cornea is generated based at least in part on the measured corneal anterior and posterior spatial dispositions and at least one corneal incision parameter. A composite image is displayed that includes an image representative of the measured corneal anterior and posterior surfaces and an image representing the corneal incision.

The present disclosure will be more fully understood from the following detailed description of the examples thereof, taken together with the drawings in which:

### BRIEF DESCRIPTION OF THE DRAWINGS

Fig. 1 is a pictorial view of a phacoemulsification system constructed to operate in accordance with an example of the present disclosure;
Figs. 2A and 2B are schematic illustrations of a graphical user interface (GUI) of the system of Fig. 1, the GUI displaying two predefined user protocols, in accordance with an example of the present disclosure; and
Fig. 3 is a flow chart describing steps providing a physician with a predefined phacoemulsification protocol on a GUI and means to modify the protocol and verify protocol compatibility, in accordance with examples of the present disclosure.

### SUMMARY OF THE INVENTION

The invention is defined in the independent claims. Further embodiments are defined in the dependent claims. Surgical methods disclosed herein, whilst not encompassed by the wording of the claims, are considered as useful for understanding the invention.

### DETAILED DESCRIPTION OF EXAMPLES

### OVERVIEW

A phacoemulsification system typically includes a graphical user interface (GUI), presented on a suitable Input/Output (I/O) device such as on a touchscreen, to enable a user to define a phacoemulsification protocol and modify its parameters. It is customary for an eye surgeon, just before a procedure is conducted, to customize protocol parameters using, for example, multiple tabs of the GUI displayed on such a touchscreen. However, given that an eye surgery system may be used by many surgeons, such repeated customizations are time consuming and prone to errors.

As an example of protocol customization, a phacoemulsification I/O may define needle vibration, including a number of possible different trajectories, e.g., longitudinal, planar (e.g., elliptical, circular), torsional, as well as in combinations and sub-combinations of such trajectories. In addition, the user may desire to use sequences of such trajectories. Another example of customization includes vibration driving waveform and power, and, as other examples, aspiration rate and vacuum level.

Examples of the present disclosure that are described herein provide methods and apparatus that enable defining (e.g., generating) one or more eye surgery protocols on an eye surgery system and/or uploading one or more user defined eye surgery protocols to the eye surgery system, and compatibility testing of the defined (e.g., generated) and/or predefined protocols with the system, as well as testing compatibility among the parameters of each user protocol. The disclosed techniques retest such compatibilities subsequent to user modification of one or more parameters of a predefined protocol, e.g., after a protocol is uploaded to the system and/or GUI. The one or more user defined eye surgery protocols can be uploaded to an eye surgery system from a remote location.

In one example, the disclosed technique provides a method for a user to store, prior to a procedure, a protocol comprising possible needle trajectory sequences. The method checks if a set of sequences requested by the user is compatible with working parameters of the phacoemulsification device, and, in the event of incompatibility, provides an indication to the user how to correct the incompatibility. More generally, the disclosed solution provides means to test any protocol parameter, such as aspiration rate, vacuum level and driving waveform parameters (e.g., frequency and pulse width), among others.

The disclosed method and algorithms can be provided in a form of a computer software product comprising a tangible non-transitory computer-readable medium in which program instructions are stored, and, when read by a processor, cause the processor to (i) configure a graphical user interface (GUI) to display one or more user defined eye surgery protocols, (ii) upload the one or more user defined eye surgery protocols to the system and/or GUI, (iii) test compatibility of each protocol parameter among themselves and with the eye surgery system, and (iv) provide at least one of (a) an indication of the compatibility of the one or more protocols to a user of the eye surgery system, and (b) an indication of the incompatibility of two or more parameters of protocols, one with the other, if such are found.

By providing a method and algorithm to share predefined protocols among systems and to verify protocols automatically, an eye surgeon protocol preparation for an eye surgery (e.g., phacoemulsification) in a surgical theater environment may be made easier and more consistent.

### SYSTEM DESCRIPTION

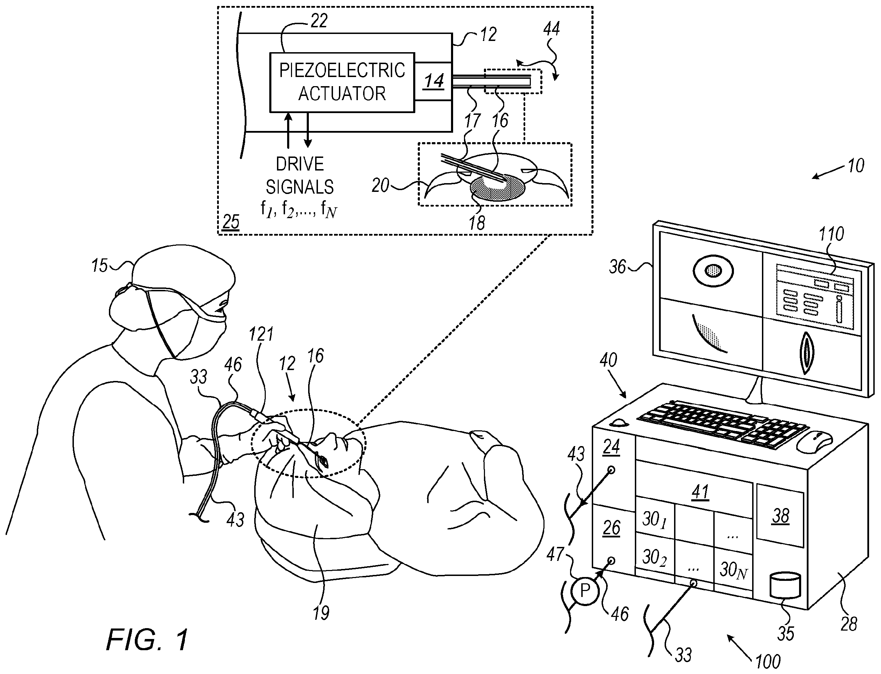

Fig. 1 is a pictorial view of a phacoemulsification system 10 constructed to operate in accordance with an example of the present disclosure. Fig. 1 includes an inset 25, and, as shown in the figure and the inset system 10, it includes a phacoemulsification probe/handpiece 12 comprising a needle 16. Needle 16 is configured to be inserted into a lens capsule 18 of an eye 20 of a patient 19. Needle 16 is mounted on a horn 14 of probe 12, and is shown in inset 25 as a straight needle. However, any suitable needle may be used with the phacoemulsification probe 12, for example, a curved or bent tip needle commercially available from Johnson & Johnson Surgical Vision, Irvine, CA, USA. A physician 15 holds handpiece 12 by a handle 121 so as to perform a phacoemulsification procedure on the eye of patient 19. The physician may activate the handpiece using a foot pedal, which is not illustrated in Fig. 1.

Handpiece 12 comprises a piezoelectric actuator 22, which is configured to vibrate horn 14 and needle 16 in one or more resonant vibration modes of the combined horn and needle element. During the phacoemulsification procedure, the vibration of needle 16 is used to break a cataract into small pieces.

During the phacoemulsification procedure, an irrigation sub-system 24, which may be located in a console 28, pumps irrigation fluid from an irrigation reservoir to an irrigation sleeve 17 that surrounds needle 16, so as to irrigate the eye. The fluid is pumped via a tubing line 43 running from the console 28 to the probe 12. Irrigation sub-system 24 is described in more detail below.

An aspiration sub-system 26, also typically located in console 28, aspirates eye fluid and waste matter (e.g., emulsified parts of the cataract) from the patient's eye via needle 16 to a collection receptacle (not shown). Aspiration sub-system 26 comprises a pump which produces a vacuum that is connected from the sub-system to probe 12 by a vacuum tubing line 46. A gauge or sensor 47 in line 46 measures the aspiration vacuum pressure. Gauge 47 may be in any convenient location in line 46, including, but not limited to, a location in or in proximity to handpiece 12 or a location in or in proximity to the console.

Irrigation sub-system 24 and aspiration sub-system 26 are both under control of a processor 38. The processor controls the volume rate of flow at which the irrigation sub-system pumps fluid. The processor also controls the vacuum pressure produced by the aspiration sub-system, using a pressure reading from gauge 47.

Some or all of the functions of processor 38 may be combined in a single physical component or, alternatively, implemented using multiple physical components. The physical components may comprise hard-wired or programmable devices, or a combination of the two. In some examples, at least some of the functions of processor 38 may be carried out by suitable software stored in a memory 35. The software may be downloaded to a device in electronic form, over a network, for example. Alternatively, or additionally, the software may be stored in tangible, non-transitory computer-readable storage media, such as optical, magnetic, or electronic memory.

Processor 38 may receive user-based commands via a user interface 40, which may include setting and/or adjusting a vibration mode and/or a frequency of piezoelectric actuator 22, setting and/or adjusting a stroke amplitude of needle 16, and setting and/or adjusting an irrigation rate, an aspiration rate, and vacuum levels of irrigation sub-system 24 and aspiration sub-system 26. Additionally, or alternatively, processor 38 may receive user-based commands from controls located in handpiece 12, to, for example, select a trajectory 44, or another trajectory, for needle 16. The implementation of a trajectory such as trajectory 44 is described further below.

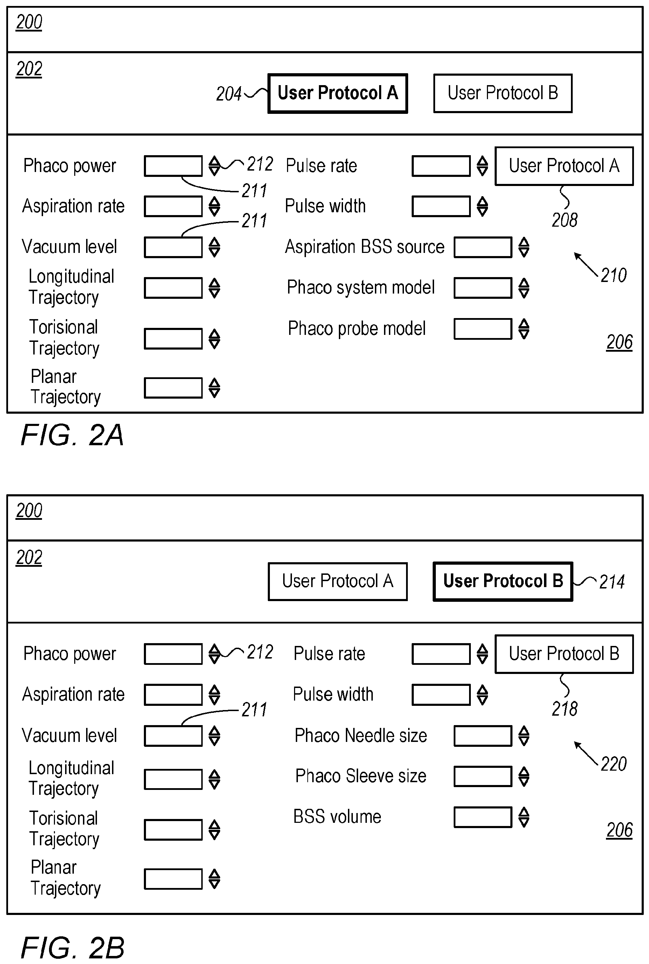

Processor 38 may present a user protocol 110 (the likes of described in detail in Fig. 2), and results of the phacoemulsification procedure on a display 36. User protocol 110 may be uploaded to the system from a remote location or be defined (e.g., generated) by the user locally, on the system, using an I/O device of the system. In an example, user interface 40 and display 36 may be one and the same, such as a touch screen graphical user interface (GUI). More generally, user interface 40 and display 36 are regarded as an example of an I/O device that presents the disclosed GUI. Processor 38 may present the disclosed graphical user interface (including both displaying GUI elements and receiving user input) using any other suitable I/O device.

The procedure illustrated in Fig. 1 may include further elements, which are omitted for clarity of presentation. For example, physician 15 typically performs the procedure using a stereo-microscope or magnifying glasses, neither of which are shown. Physician 15 may use other surgical tools, in addition to probe 12, which are also not shown to maintain clarity and simplicity.

Console 28 further comprises a multi-channel piezoelectric drive system 100 comprising drive-modules 30₁, 30₂, ... 30_{N}, each coupled, using wiring in a cable 33, with a stack of piezoelectric crystals of actuator 22. Drive-modules 30₁, 30₂, ... 30_{N}, generically termed drive-modules 30, are controlled by processor 38 and convey phase-controlled driving signals via cable 33 to piezoelectric actuator 22. In response, piezoelectric actuator 22 vibrates needle 16, which performs a vibrational/ultrasound trajectory 44, the trajectory typically comprising for example, one or a combination of the following: longitudinal, transverse, and/or torsional ultrasonic vibrations, which may be operated in synchronization one with the other. System 100 is described further below, with reference to Figs. 2A and 2B.

### MANAGING PHACOEMULSIFICATION USER DEFINED PROTOCOLS

Figs. 2A and 2B show two schematic illustrations of a graphical user interface (GUI) 200 of system 10 of Fig. 1, the GUI displaying two predefined user protocols (210, 220), in accordance with an example of the present disclosure.

As seen in the example, GUI 200 includes a region 202 that displays, using a highlighted tab (tab 204 in Fig. 2A and tab 214 in Fig. 2B), the active (e.g., editable) protocol. An active protocol (i.e., Protocol A or Protocol B) is further titled by respective tabs 208 and 218 in region 206 of the GUI that comprises parameter fields 211 (e.g., entries).

As seen, parameter fields 211 of both of the different protocols 210 and 220 can be modified using, for example, arrows 212 to change (e.g., increase or decrease) a parameter value. In an alternate example, the parameter fields may be entered using a keyboard (physical as seen Fig. 1 or virtual).

The user can define a protocol by, for exmaple, setting parameter values in predefined fields 211 using arrows 212 to increase or decrease any of the values. The user may further define a protocol by adding one or more fields taken from a predefined list of fields available in software for generating a protocol.

The shown GUI is configured to provide an indication of the protocol compatibility to a user of the eye surgery system by, for example, changing a color of the tab between green (for "compatible") to red (for "incompatible"), and further highlighting fields 211 (e.g., titles) of incompatible parameters.

In addition, the GUI highlights parameters that are incompatible with each other, providing an indication of the incompatibility to a user of the phacoemulsification system.

The example illustrations shown in Figs. 2A and 2B are chosen purely for the sake of conceptual clarity. Figs. 2A and 2B show only parts relevant to examples of the present disclosure.

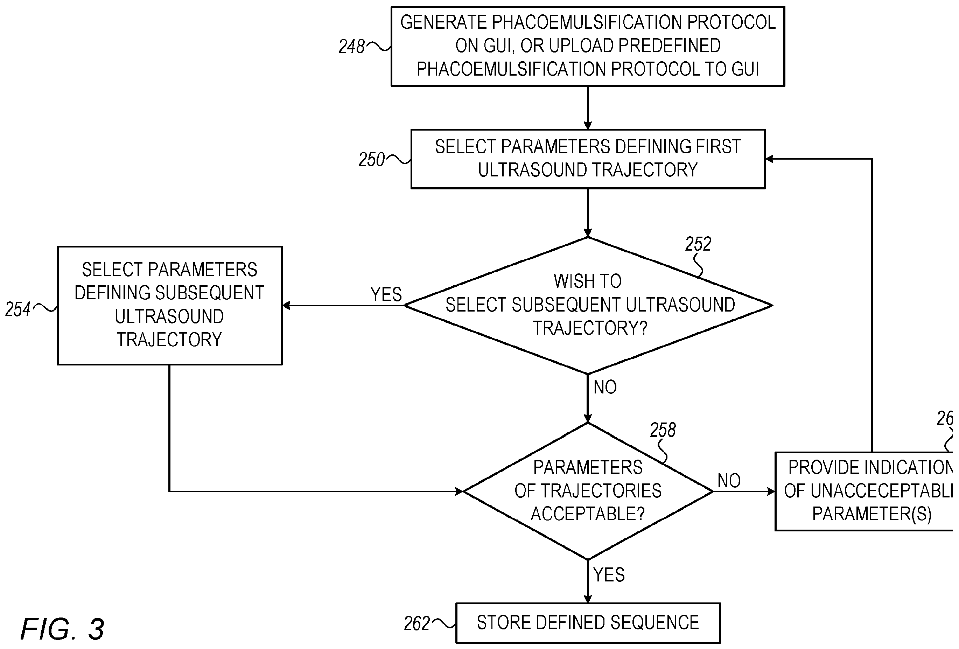

Fig. 3 is a flow chart describing steps providing a physician with a predefined phacoemulsification protocol on GUI 200 and means to modify the protocol and verify protocol compatibility, in accordance with examples of the present disclosure.

For example, during a phacoemulsification procedure, there are a large number of parameters that the physician performing the procedure may select, in order to control the ultrasonic trajectory of needle 16. In addition to controlling the trajectory, i.e., the path followed by a distal end of the needle, the physician also controls the power delivered (which typically affects the amplitude of the ultrasonic vibration) as well as the duration of the trajectory.

The flow chart of Fig. 3 describes steps that simplify the choice of parameters to be made by physician 15 in setting ultrasound trajectories for needle 16, in accordance with an example of the present invention. The flow chart assumes that the physician prepares, prior to a procedure, one or more trajectory sequences, each sequence comprising two or more individual trajectories. The physician typically uses a dedicated GUI tab for presenting, preparing, and/or modifying the sequences. During the procedure, the sequences are presented to the physician on display 36, and the physician may, for example, select a sequence from the display.

In an initial step 248 of the flow chart, the physician may program its own protocol using an I/O device, upload a predefined protocol (e.g., from a remote location) or select one or more predefined phacoemulsification protocols to/from GUI 200. Such protocols include, for example, parameters defining a trajectory of the vibrating needle 16 of phacoemulsification handpiece 12.

In a subsequent step 250 of the flow chart, the physician selects parameters defining a first ultrasound trajectory. The parameters typically include if the trajectory is to be longitudinal, planar (e.g., elliptical, or circular), or torsional, the power to be applied, and the duration of the trajectory. The trajectories may also comprise a combination of longitudinal, planar, or torsional trajectories, in which case the physician provides an amplitude for each of the different types or ratios of the powers for each of the different types, as well as an overall power for the combination.

Processor 38 translates the defined parameters into inputs to be provided by the processor to drive-modules 30.

Next, at a checking step 252, the processor checks if the physician wishes to select parameters defining a subsequent ultrasound trajectory. If the answer is no, the process goes directly to step 258.

If the answer is yes, in a subsequent step 254, the physician selects parameters defining a subsequent ultrasound trajectory, substantially as described for step 250. The physician may also select a transition period between the trajectories of steps 250 and 254. As for step 250, processor 38 translates the parameters into inputs for drive-modules 30.

If appropriate, the physician may iterate step 254 to define a further subsequent trajectory.

In a decision step 258 processor 38 evaluates if the parameters of the trajectories selected in steps 250 and 254 are acceptable, i.e., are within the capabilities of drive-modules 30, or are mutually compatible. The processor may evaluate the parameters selected in steps 250 and 254 against allowed parameter ranges stored in a look-up table, or using an algorithm, to determine if the selected parameters are indeed acceptable or not.

If step 258 returns positive ("Yes"), i.e., the sequence defined in steps 250 and 254 is acceptable, control continues to a final storage step 262, wherein the processor stores the defined sequence. The stored sequence may be presented to the physician on display 36 for selection during a phacoemulsification procedure.

If step 258 returns negative ("No"), i.e., the sequence defined in steps 250 and 254 is not acceptable, control continues to a notification step 266, wherein the processor provides the physician with an indication of what is unacceptable in the selected sequence. For example, while transitions between different sequences may typically be defined with a resolution of 20 µs, the physician may have requested a faster transition. In this case the processor may indicate to the physician that 20 µs is the minimum possible transition period.

From step 266 control returns to the beginning of the flow chart, so that the physician can change the definitions of selected sequences.

As stated above, physician 15 can use the flow chart to define acceptable sets of sequences. One sequence, according to an example of the present invention, comprises a first trajectory that is a longitudinal vibration, with a subsequent trajectory that is an elliptical vibration. Operating with a longitudinal mode is efficient in cutting lens material, but it may produce propagation waves that may damage other tissue. Use of a subsequent elliptical vibration may reduce formation of propagation waves.

The example flow chart shown in Fig. 3 is chosen purely for the sake of conceptual clarity. For example, other aforementioned parameters are tested and can be modified on GUI 200, such as irrigation, aspiration, vacuum, and waveform parameters, which are not considered here for simplicity and clarity of presentation.

Although the examples described herein mainly address phacoemulsification, the methods and systems described herein can also be used in other eye surgical applications that can benefit of user predefined protocols, such as Vitrectomy.

It will thus be appreciated that the examples described above are cited by way of example, and that the present disclosure is not limited to what has been particularly shown and described hereinabove. Rather, the present disclosure includes both combinations and sub-combinations of the various features described hereinabove, as well as variations and modifications thereof which would occur to persons skilled in the art upon reading the foregoing description and which are not disclosed in the prior art. The scope of the invention is defined by the appended claims.

## Claims

1. An eye surgery system (10), comprising:
an Input/Output, I/O, device (36, 40), configured to enable a user (15) to define one or more eye surgery protocols, and further configured to, using the I/O device, present a graphical user interface, GUI (200), that displays the one or more user defined eye surgery protocols (204, 208); and
a processor (38), which is configured to:
present the one or more user defined eye surgery protocols on the I/O device using the GUI;
test compatibility of the one or more user defined eye surgery protocols with the eye surgery system; and
provide an indication of the compatibility to a user of the eye surgery system;
wherein providing the indication of the compatibility comprises highlighting parameters of the user defined eye surgery protocols that are incompatible with each other.

2. The system according to claim 1, wherein the processor is configured to present the one or more user defined protocols by uploading the one or more user defined eye surgery protocols to the GUI.

3. The system according to any one of claim 1 to claim 2, wherein the processor is further configured to indicate to the user an incompatibility between parameters of a user defined eye surgery protocol.

4. The system according to any one of claim 1 to claim 3, wherein the processor is further configured to enable the user to modify a parameter of the one or more user defined eye surgery protocols using the GUI, to test the compatibility of the modified parameter with the eye surgery system, and to provide an indication of the compatibility to the user.

5. A method, comprising:
defining one or more eye surgery protocols (204, 208) using an Input/Output, I/O, device (36, 40), and, using the I/O device, presenting a graphical user interface, GUI (200), that is configured to display the one or more user defined eye surgery protocols;
presenting, with a processor (38), the one or more user defined eye surgery protocols on the I/O device using the GUI;
testing, with the processor, compatibility of the one or more user defined eye surgery protocols with the eye surgery system; and
providing, with the processor, an indication of the compatibility to a user (15) of the eye surgery system;
wherein providing the indication of the compatibility comprises highlighting parameters of the user defined eye surgery protocols that are incompatible with each other.

6. The method according to claim 5, wherein presenting the one or more user defined protocols comprises uploading the one or more user defined eye surgery protocols to the GUI.

7. The system according to any one of claim 1 or claim 2 or the method according to claim 6, wherein the one or more user defined eye surgery protocols are uploaded from a remote location.

8. The method according to claim 5, further comprising indicating to the user an incompatibility between parameters of a user defined eye surgery protocol.

9. The method according to claim 5, further comprising enabling the user to modify a parameter of the one or more user defined eye surgery protocols using the GUI, to test the compatibility of the modified parameter with the eye surgery system, and to provide an indication of the compatibility to the user.

10. The system according to any one of claims 1 to 4 or claim 7 or the method according to claim 5, wherein the eye surgery system comprises a phacoemulsification system and the protocols comprise one or more phacoemulsification protocols.

11. The system according to claim 10 or the method according to claim 10, wherein the phacoemulsification system comprises:
a phacoemulsification handpiece (12) comprising one or more piezoelectric crystals configured to vibrate a needle (16) coupled with the one or more piezoelectric crystals; and
one or more drive-modules (30₁, 30₂, 30_{N}) configured to vibrate the one or more piezoelectric crystals in a plurality of trajectories, in response to respective inputs to the drive-modules,
wherein the processor is configured to:
select first parameters configured to vibrate the needle in a first trajectory;
select second parameters configured to vibrate the needle in a second trajectory;
determine that the inputs required for the drive-modules, in response to the first and second parameters, are incompatible with each other; and
provide an indication of the incompatibility to the user.

12. The system according to claim 10 or the method according to claim 10, wherein the one or more phacoemulsification protocols specify parameters comprising one or more of: aspiration rate, vacuum level, phacoemulsification power and driving waveform parameters.

13. The system according to any one of claim 1 to claim 4, claim 7, or claim 10, wherein the processor is configured to provide the indication of the incompatibility by highlighting one or more fields (211) of the GUI.

14. The method according to any one of claim 5 or claim 10, wherein providing the indication of the incompatibility comprises highlighting one or more fields (211) of the GUI.

15. A computer software product, the product comprising a tangible non-transitory computer-readable medium in which program instructions are stored, which instructions, when read by a processor (38), cause the processor to:
enable a user (15) to define one or more eye surgery protocols using an Input/Output, I/O, device (36, 40), and, using the I/O device, to present a graphical user interface, GUI (200), that displays one or more user defined eye surgery protocols;
present the one or more user defined eye surgery protocols on the I/O device using the GUI;
test compatibility of the one or more user defined eye surgery protocols with an eye surgery system (10); and
provide an indication of the compatibility to a user of the eye surgery system;
wherein providing the indication of the compatibility comprises highlighting parameters of the user defined eye surgery protocols that are incompatible with each other.

## Patentansprüche

1. Augenchirurgiesystem (10), umfassend:
eine Eingabe-/Ausgabe-, E/A-, Vorrichtung (36, 40), die konfiguriert ist, um einem Benutzer (15) zu ermöglichen, ein oder mehrere Augenchirurgieprotokolle zu definieren, und ferner konfiguriert ist, um unter Verwendung der E/A-Vorrichtung eine grafische Bedienerschnittstelle, GUI (200), darzustellen, die das eine oder die mehreren benutzerdefinierten Augenchirurgieprotokolle (204, 208) anzeigt; und
einen Prozessor (38), der konfiguriert ist zum:
Darstellen des einen oder der mehreren benutzerdefinierten Augenchirurgieprotokolle auf der E/A-Vorrichtung unter Verwendung der GUI;
Testen der Kompatibilität des einen oder der mehreren benutzerdefinierten Augenchirurgieprotokolle mit dem Augenchirurgiesystem; und
Bereitstellen einer Anzeige der Kompatibilität für einen Benutzer des Augenchirurgiesystems;
wobei das Bereitstellen der Anzeige der Kompatibilität das Hervorheben von Parametern der benutzerdefinierten Augenchirurgieprotokolle umfasst, die miteinander inkompatibel sind.

2. System nach Anspruch 1, wobei der Prozessor konfiguriert ist, um die ein oder mehreren benutzerdefinierten Protokolle durch Hochladen der ein oder mehreren benutzerdefinierten Augenchirurgieprotokolle auf die GUI darzustellen.

3. System nach einem der Ansprüche 1 bis 2, wobei der Prozessor ferner konfiguriert ist, um dem Benutzer eine Inkompatibilität zwischen Parametern eines benutzerdefinierten Augenchirurgieprotokolls anzuzeigen.

4. System nach einem der Ansprüche 1 bis 3, wobei der Prozessor ferner konfiguriert ist, um dem Benutzer zu ermöglichen, einen Parameter des einen oder der mehreren benutzerdefinierten Augenchirurgieprotokolle unter Verwendung der GUI zu modifizieren, um die Kompatibilität des modifizierten Parameters mit dem Augenchirurgiesystem zu testen, und um dem Benutzer eine Anzeige der Kompatibilität bereitzustellen.

5. Verfahren, umfassend:
Definieren eines oder mehrerer Augenchirurgieprotokolle (204, 208) unter Verwendung einer Eingabe-/Ausgabe-, I/O-, Vorrichtung (36, 40), und, unter Verwendung der I/O-Vorrichtung, Darstellen einer grafischen Bedienerschnittstelle, GUI (200), die konfiguriert ist, um das eine oder die mehreren benutzerdefinierten Augenchirurgieprotokolle anzuzeigen;
Darstellen der einen oder mehreren benutzerdefinierten Augenchirurgieprotokolle auf der E/A-Vorrichtung unter Verwendung der GUI mit einem Prozessor (38);
Testen, mit dem Prozessor, der Kompatibilität des einen oder der mehreren benutzerdefinierten Augenchirurgieprotokolle mit dem Augenchirurgiesystem; und
Bereitstellen einer Anzeige der Kompatibilität für einen Benutzer (15) des Augenchirurgiesystems mit dem Prozessor;
wobei das Bereitstellen der Anzeige der Kompatibilität das Hervorheben von Parametern der benutzerdefinierten Augenchirurgieprotokolle umfasst, die miteinander inkompatibel sind.

6. Verfahren nach Anspruch 5, wobei das Darstellen des einen oder der mehreren benutzerdefinierten Protokolle das Hochladen des einen oder der mehreren benutzerdefinierten Augenchirurgieprotokolle auf die GUI umfasst.

7. System nach einem der Ansprüche 1 oder 2 oder Verfahren nach Anspruch 6, wobei die ein oder mehreren benutzerdefinierten Augenchirurgieprotokolle von einem entfernten Ort hochgeladen werden.

8. Verfahren nach Anspruch 5, ferner umfassend das Anzeigen einer Inkompatibilität zwischen Parametern eines benutzerdefinierten Augenchirurgieprotokolls an den Benutzer.

9. Verfahren nach Anspruch 5, ferner umfassend das Ermöglichen, dass der Benutzer einen Parameter des einen oder der mehreren benutzerdefinierten Augenchirurgieprotokolle unter Verwendung der GUI modifiziert, die Kompatibilität des modifizierten Parameters mit dem Augenchirurgiesystem testet und dem Benutzer eine Anzeige der Kompatibilität bereitstellt.

10. System nach einem der Ansprüche 1 bis 4 oder Anspruch 7 oder das Verfahren nach Anspruch 5, wobei das Augenchirurgiesystem ein Phakoemulsifikationssystem umfasst und die Protokolle ein oder mehrere Phakoemulsifikationsprotokolle umfassen.

11. System nach Anspruch 10 oder Verfahren nach Anspruch 10, wobei das Phakoemulsifikationssystem umfasst:
ein Phakoemulsifikationshandstück (12), das einen oder mehrere piezoelektrische Kristalle umfasst, die konfiguriert sind, um eine Nadel (16) in Schwingung zu versetzen, die mit dem einen oder den mehreren piezoelektrischen Kristallen gekoppelt ist; und
ein oder mehrere Antriebsmodule (30₁, 30₂, 30_{N}), die konfiguriert sind, um den einen oder die mehreren piezoelektrischen Kristalle in einer Vielzahl von Trajektorien in Schwingung zu versetzen, als Reaktion auf entsprechende Eingaben an die Antriebsmodule,
wobei der Prozessor konfiguriert ist zum:
Auswählen erster Parameter, die konfiguriert sind, um die Nadel in einer ersten Trajektorie in Schwingung zu versetzen;
Auswählen zweiter Parameter, die konfiguriert sind, um die Nadel in einer zweiten Trajektorie in Schwingung zu versetzen;
Bestimmen, dass die für die Antriebsmodule erforderlichen Eingaben als Reaktion auf die ersten und zweiten Parameter miteinander inkompatibel sind; und
Bereitstellen einer Anzeige der Inkompatibilität für den Benutzer.

12. System nach Anspruch 10 oder Verfahren nach Anspruch 10, wobei das eine oder die mehreren Phakoemulsifikationsprotokolle Parameter spezifizieren, die eines oder mehrere der folgenden umfassen: Aspirationsrate, Vakuumniveau, Phakoemulsifikationsleistung und Antriebswellenformparameter.

13. System nach einem der Ansprüche 1 bis 4, Anspruch 7 oder Anspruch 10, wobei der Prozessor konfiguriert ist, um die Anzeige der Inkompatibilität durch Hervorheben eines oder mehrerer Felder (211) der GUI bereitzustellen.

14. Verfahren nach einem der Ansprüche 5 oder 10, wobei das Bereitstellen der Anzeige der Inkompatibilität das Hervorheben eines oder mehrerer Felder (211) der GUI umfasst.

15. Computersoftwareprodukt, das Produkt umfassend ein greifbares, nicht transitorisches, computerlesbares Medium, auf dem Programmanweisungen gespeichert sind, wobei die Anweisungen, wenn sie durch einen Prozessor (38) ausgeführt werden, den Prozessor veranlassen zum:
Ermöglichen für einen Benutzer (15), ein oder mehrere Augenchirurgieprotokolle unter Verwendung einer Eingabe-/Ausgabe-, I/O-, Vorrichtung (36, 40) zu definieren, und unter Verwendung der I/O-Vorrichtung eine grafische Bedienerschnittstelle, GUI (200), darzustellen, die ein oder mehrere benutzerdefinierte Augenchirurgieprotokolle anzeigt;
Darstellen des einen oder der mehreren benutzerdefinierten Augenchirurgieprotokolle auf der E/A-Vorrichtung unter Verwendung der GUI;
Testen der Kompatibilität des einen oder der mehreren benutzerdefinierten Augenchirurgieprotokolle mit einem Augenchirurgiesystem (10); und
Bereitstellen einer Anzeige der Kompatibilität für einen Benutzer des Augenchirurgiesystems;
wobei das Bereitstellen der Anzeige der Kompatibilität das Hervorheben von Parametern der benutzerdefinierten Augenchirurgieprotokolle umfasst, die miteinander inkompatibel sind.

## Revendications

1. Système de chirurgie oculaire (10), comprenant :
un dispositif d'entrée/sortie, E/S (36, 40), configuré pour permettre à un utilisateur (15) de définir un ou plusieurs protocoles de chirurgie oculaire, et configuré en outre, à l'aide du dispositif d'E/S, pour présenter une interface graphique utilisateur, GUI (200), qui affiche le ou les protocoles de chirurgie oculaire (204, 208) définis par l'utilisateur ; et
un processeur (38) qui est configuré pour :
présenter le ou les protocoles de chirurgie oculaire définis par l'utilisateur sur le dispositif d'E/S à l'aide de la GUI ;
tester la compatibilité du ou des protocoles de chirurgie oculaire définis par l'utilisateur avec le système de chirurgie oculaire ; et
fournir une indication de la compatibilité à un utilisateur du système de chirurgie oculaire ;
dans lequel la fourniture de l'indication de la compatibilité comprend la mise en évidence de paramètres des protocoles de chirurgie oculaire définis par l'utilisateur qui sont incompatibles les uns avec les autres.

2. Système selon la revendication 1, dans lequel le processeur est configuré pour présenter le ou les protocoles définis par l'utilisateur en téléversant le ou les protocoles de chirurgie oculaire définis par l'utilisateur vers la GUI.

3. Système selon l'une quelconque de la revendication 1 à la revendication 2, dans lequel le processeur est configuré en outre pour indiquer à l'utilisateur une incompatibilité entre des paramètres d'un protocole de chirurgie oculaire défini par l'utilisateur.

4. Système selon l'une quelconque de la revendication 1 à la revendication 3, dans lequel le processeur est configuré en outre pour permettre à l'utilisateur de modifier un paramètre du ou des protocoles de chirurgie oculaire définis par l'utilisateur à l'aide de la GUI, pour tester la compatibilité du paramètre modifié avec le système de chirurgie oculaire, et pour fournir une indication de la compatibilité à l'utilisateur.

5. Procédé, comprenant :
la définition d'un ou plusieurs protocoles de chirurgie oculaire (204, 208) à l'aide d'un dispositif d'entrée/sortie, E/S (36, 40), et, à l'aide du dispositif d'E/S, la présentation d'une interface graphique utilisateur, GUI (200), qui est configurée pour afficher le ou les protocoles de chirurgie oculaire définis par l'utilisateur ;
la présentation, avec un processeur (38), du ou des protocoles de chirurgie oculaire définis par l'utilisateur sur le dispositif d'E/S à l'aide de la GUI ;
le fait de tester, avec le processeur, la compatibilité du ou des protocoles de chirurgie oculaire définis par l'utilisateur avec le système de chirurgie oculaire ; et
la fourniture, avec le processeur, d'une indication de la compatibilité à un utilisateur (15) du système de chirurgie oculaire ;
dans lequel la fourniture de l'indication de la compatibilité comprend la mise en évidence de paramètres des protocoles de chirurgie oculaire définis par l'utilisateur qui sont incompatibles les uns avec les autres.

6. Procédé selon la revendication 5, dans lequel la présentation du ou des protocoles définis par l'utilisateur comprend le téléversement du ou des protocoles de chirurgie oculaire définis par l'utilisateur vers la GUI.

7. Système selon l'une quelconque de la revendication 1 ou de la revendication 2 ou procédé selon la revendication 6, dans lequel le ou les protocoles de chirurgie oculaire définis par l'utilisateur sont téléversés à partir d'un emplacement distant.

8. Procédé selon la revendication 5, comprenant en outre le fait d'indiquer à l'utilisateur une incompatibilité entre des paramètres d'un protocole de chirurgie oculaire défini par l'utilisateur.

9. Procédé selon la revendication 5, comprenant en outre le fait de permettre à l'utilisateur de modifier un paramètre du ou des protocoles de chirurgie oculaire définis par l'utilisateur à l'aide de la GUI, pour tester la compatibilité du paramètre modifié avec le système de chirurgie oculaire, et pour fournir une indication de la compatibilité à l'utilisateur.

10. Système selon l'une quelconque des revendications 1 à 4 ou la revendication 7 ou procédé selon la revendication 5, dans lequel le système de chirurgie oculaire comprend un système de phacoémulsification et les protocoles comprennent un ou plusieurs protocoles de phacoémulsification.

11. Système selon la revendication 10 ou procédé selon la revendication 10, dans lequel le système de phacoémulsification comprend :
une pièce à main de phacoémulsification (12) comprenant un ou plusieurs cristaux piézoélectriques conçus pour mettre en vibration une aiguille (16) accouplée à un ou plusieurs cristaux piézoélectriques ; et
un ou plusieurs modules de pilotage (30₁, 30₂, 30_{N}) configurés pour mettre en vibration le ou les cristaux piézoélectriques dans une pluralité de trajectoires, en réponse à des entrées respectives aux modules de pilotage,
dans lequel le processeur est configuré pour :
sélectionner des premiers paramètres configurés pour mettre en vibration l'aiguille dans une première trajectoire ;
sélectionner des seconds paramètres configurés pour mettre en vibration l'aiguille dans une seconde trajectoire ;
déterminer que les entrées requises pour les modules de pilotage, en réponse aux premier et second paramètres, sont incompatibles les unes avec les autres ; et
fournir une indication de l'incompatibilité à l'utilisateur.

12. Système selon la revendication 10 ou procédé selon la revendication 10, dans lequel le ou les protocoles de phacoémulsification spécifient des paramètres comprenant un ou plusieurs parmi : fréquence d'aspiration, niveau de vide, puissance de phacoémulsification et paramètres de forme d'onde de pilotage.

13. Système selon l'une quelconque de la revendication 1 à la revendication 4, de la revendication 7, ou de la revendication 10, dans lequel le processeur est configuré pour fournir l'indication de l'incompatibilité par mise en évidence d'un ou plusieurs champs (211) de la GUI.

14. Procédé selon l'une quelconque de la revendication 5 ou de la revendication 10, dans lequel la fourniture de l'indication de l'incompatibilité comprend la mise en évidence d'un ou plusieurs champs (211) de la GUI.

15. Produit logiciel d'ordinateur, le produit comprenant un support tangible non transitoire lisible par ordinateur dans lequel sont stockées des instructions de programme, ces instructions, lorsqu'elles sont exécutées par un processeur (38), amenant le processeur à :
permettre à un utilisateur (15) de définir un ou plusieurs protocoles de chirurgie oculaire à l'aide d'un dispositif d'entrée/sortie, E/S (36, 40), et, à l'aide du dispositif d'E/S, de présenter une interface graphique utilisateur, GUI (200), qui affiche un ou plusieurs protocoles de chirurgie oculaire définis par l'utilisateur ;
présenter le ou les protocoles de chirurgie oculaire définis par l'utilisateur sur le dispositif d'E/S à l'aide de la GUI ;
tester la compatibilité du ou des protocoles de chirurgie oculaire définis par l'utilisateur avec un système de chirurgie oculaire (10) ; et
fournir une indication de la compatibilité à un utilisateur du système de chirurgie oculaire ;
dans lequel la fourniture de l'indication de la compatibilité comprend la mise en évidence de paramètres des protocoles de chirurgie oculaire définis par l'utilisateur qui sont incompatibles les uns avec les autres.
